# EUROPEAN PATENT SPECIFICATION

(11) **EP 1 284 667 B1**
(45) Date of publication and mention of the grant of the patent: **22.02.2006**
(21) Application number: 01935889.4
(22) Date of filing: 25.05.2001
(51) Int. Cl.: A61B 17/70, A61B 17/86

(54) **ANCHORING SYSTEM FOR FIXING OBJECTS TO BONES**
VERANKERUNGSSYSTEM ZUM BEFESTIGEN VON GEGENSTÄNDEN AN KNOCHEN
SYSTEME DE FIXATION PERMETTANT DE FIXER DES OBJETS SUR DES OS

(30) Priority: 25.05.2000 US 206811 P
(43) Date of publication of application: 26.02.2003
(73) Proprietor: Orthoplex LLC, Boston, Massachusetts 02110 (US)
(72) Inventor: Sevrain, Lionel C., Plymouth, MA 02360 (US)
(74) Representative: McKechnie, Neil Henry
(86) International application number: PCT/CA2001/000751
(87) International publication number: WO 2001/089400

(56) References cited:
- US-A- 5 480 402
- US-A- 5 514 137
- US-A- 5 603 713
- US-A- 5 800 433

## Description

### BACKGROUND OF THE INVENTION

### (a) Field of the Invention

The present invention relates to devices for attaching various objects, such as prostheses or implants, to bones, including for anchoring spinal instrumentations to vertebrae of the human rachis.

### (b) Description of Prior Art

U.S. Patents No. 5,366,455 issued to Dove et al. on November 22, 1994, No. 5,672,175 issued to Martin on September 30, 1997, No. 5,733,284 issued to Martin on March 31, 1998 and No. 5,437,672 issued to Alleyne on August 1, 1995 disclose devices for anchoring various supports, e.g. spinal orthoses, to the rachis, these devices being adapted to obviously extend outwardly of the spinous process or canal and thus of the spinal cord.

U.S. Patents No. 5,800,433 issued to Benzel et al. on September 1, 1998 and No. 5,954,722 issued to Bono on September 21, 1999 teach anchoring systems having screws which are angled such as to converge towards each other.

U.S. Patents No. 5,904,683 issued to Pohndorf et al. on May 18, 1999 and No.5, 980,523 issued to Jackson on November 9, 1999 disclose anterior cervical vertebral stabilising devices held in place by various types of screws.

To try preventing the screws from loosening, various systems have been used, such as directing the screws along different orientations (e.g. diverging or converging); providing a locking mechanism on the screw (e.g. counter-nut); modifying the screw's thread (height and depth); engaging each screw to two tissues having different densities; etc.

For example, US Patent No. 5,603,713 to Aust et al discloses an anterior lumbar fixation device, which is designed to capture two cortical surfaces via screw insertion. The fact that the screws contact the cortical surfaces serves to strengthen the connection to the bone and to reduce the length of screw required.

Alternative solutions for other bones and joints are provided, for example by US Patent No. 5,480,402 and 5,514,137 which disclose a compression interlocking system for stabilising shoulder fractures. US Patent No. 5,514,137 discloses a variety of methods of fixing orthopaedic devices within bones, including temporary fixations for the duration of repairs to fractures, etc.

### SUMMARY OF THE INVENTION

It is therefore an aim of the present invention to provide a novel anchoring system for securing various objects to bones, such as spinal devices or instrumentations to the rachis.

It is also an aim of the present invention to provide an anchoring system well adapted to prevent a loosening thereof over time.

Therefore, in accordance with the present invention, there is provided an anchoring system for mounting an object to a bone, comprising first and second anchoring members each having proximal and distal ends, said proximal ends being adapted to hold the object to the bone while said distal ends are in the bone, characterised in that said proximal ends are spaced from each other and said first and second anchoring members are adapted to be connected to each other in the bone.

Preferably said distal ends define co-operating engagement means for connecting said distal ends together in the bone.

Preferably co-operating engagement means comprise inter- engageable male threaded means and female threaded means provided respectively on said distal ends of said second and first anchoring members.

Preferably said male threaded means comprise a threaded section provided at said distal end of said second anchoring member, and wherein said female threaded means comprise a threaded opening obliquely defined at said distal end of said first anchoring member, said threaded section being adapted to threadably engage said threaded opening.

Preferably said first and second anchoring members comprise respectively first and second screws, said first screw defining said threaded opening.

Optionally said proximal end of said first anchoring member is provided with indicia means for indicating an orientation of said female threaded means in the bone thereby facilitating the engagement therein of said male threaded means of said second anchoring member.

Preferably said proximal ends of each of said first and second anchoring members are provided with a head engageable by a torque-producing tool for engaging said first and second anchoring members in the bone, said indicia means being at least provided on said head of said first anchoring member.

Optionally said male threaded means comprise a threaded section provided at said distal end of said second anchoring member, and wherein said female threaded means comprise a threaded opening defined through a ball rotatably mounted in a socket defined at said distal end of said first anchoring member, said threaded section being adapted to threadably engage said threaded opening.

Preferably the anchoring system further comprises a support member, said proximal ends of said first and second anchoring members being adapted to hold said support member to the bone while said distal ends are connected to each other in the bone thereby forming a triangular frame.

Preferably said support member defines a pair of holes, said first and second anchoring members being adapted to extend through said holes with said proximal ends engaging said support member adjacent said holes.

Preferably said first and second anchoring members converge from said proximal ends towards said distal ends, said distal ends defining co-operating engagement means for connecting said distal ends together in the bone.

Preferably said co-operating engagement means comprise inter- engageable male threaded means and female threaded means provided respectively on said distal ends of said second and first anchoring members.

Preferably said male threaded means comprise a threaded section provided at said distal end of said second anchoring member, and wherein said female threaded means comprise a threaded opening obliquely defined at said distal end of said first anchoring member, said threaded section being adapted to threadably engage said threaded opening.

Preferably said first and second anchoring members comprise respectively first and second screws, said first screw defining said threaded opening.

Optionally said proximal end of said first anchoring member is provided with indicia means for indicating an orientation of said female threaded means in the bone thereby facilitating the engagement therein of said male threaded means of said second anchoring member.

Optionally said co-operating engagement means comprise inter-engageable male and female means provided respectively on said distal ends of said second and first anchoring members.

Preferably said female means comprise an elongated slot and said male means include a lateral extension at said distal end of said second anchoring member, said distal end and said lateral extension of said second anchoring member being insertable through said slot, wherein upon adequate rotation of said distal end of said second anchoring member, said lateral extension becomes locked behind said distal end of said first anchoring member.

Preferably said distal end of said second anchoring member and said lateral extension are one of T-shaped, L-shaped and barb- shaped.

Optionallu said first and second anchoring members are threadless pins or nails.

Optionally said co-operating engagement means comprise a male threaded means at said distal end of said second anchoring member and an opening in said distal end of said first anchoring member adapted to be tapped by said male threaded means upon rotary engagement of said male threaded means in said opening.

Preferably said male threaded means comprise a threaded section provided at said distal end of said second anchoring member, and wherein said opening is defined in a ball rotatably mounted in a socket defined at said distal end of said first anchoring member.

### BRIEF DESCRIPTION OF THE DRAWINGS

Having thus generally described the nature of the invention, reference will now be made to the accompanying drawings, showing by way of illustration a preferred embodiment thereof, and in which:
Fig. 1 is a schematic cross-sectional plan view of a bridging plate mounted to a lumbar vertebra using an anchoring system in accordance with the present invention; and
Fig. 2 is a schematic anterior perspective view of a bridging plate mounted to a pair of cervical vertebra using the anchoring system of the present invention.

### DESCRIPTION OF THE PREFERRED EMBODIMENTS

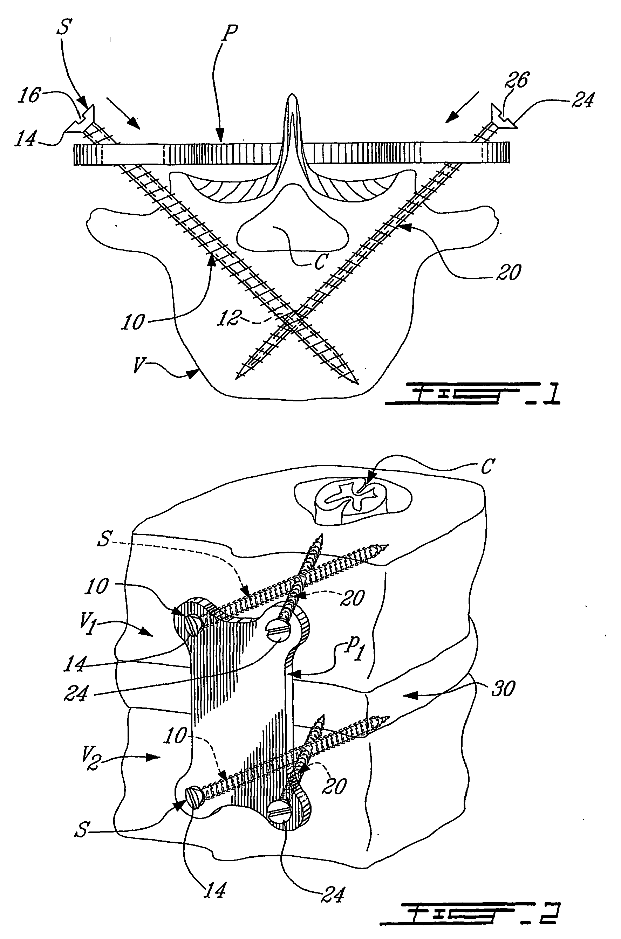

Fig. 1 illustrates an anchoring system S in accordance with the present invention which is herein schematically shown in an engaged position to a lumbar vertebra V of the human rachis for holding firmly thereagainst a spinal prosthesis or spinal instrumentation, such as a support plate P, from a posterior approach.

Fig. 2 illustrates two anchoring systems S that hold an object, such as a cervical plate P' that has been positioned after classical anterior or antero-lateral approach of the cervical spine and that is herein used to link together two or more adjacent vertebrae, such as vertebrae V₁ and V₂. For instance, when a cervical disk is anteriorly removed (see 30 in Fig. 2) from between two adjacent vertebrae, it is known to fuse both these vertebrae together to provide stability to the rachis. This can be done by securing plates to the vertebrae. To replace the disks, a spinal prosthesis may be installed and such a prosthesis typically comprises a pair of upper and lower plates secured with screws respectively to the upper and lower vertebrae between which the disk has been removed with a prosthetic disk being provided between these vertebrae and which is held in place by the plates. U.S. Patents No. 5,258,031 issued to Salib et al. on November 2, 1993 and No. 6,001,130 issued to Bryan et al. on December 14, 1999 disclose known examples of intervertebral disk prostheses which are secured to adjacent vertebrae using screws which extend therein. In these cases, one screw is used to mount each of the upper and lower support assemblies to its respective vertebra. On the other hand, U.S. Patent No. 5,755,796 issued to Ibo et al. on May 26, 1998 teaches a cervical disk prosthesis wherein each of the upper and lower plates are secured to its respective vertebra by way of a pair of screws which are horizontally spaced apart from each other. The prosthesis is secured anteriorly of the rachis and the screws thereof are short enough not to reach the spinal cord.

In other cases however (posterior approach), the screws are longer and are generally directed on each side of the spinous canal in order to obviously prevent damage to the spinal cord.

Therefore, the present anchoring system S (for each of Figs. 1 and 2) comprises first and second screws 10 and 12, respectively, which are adapted to be introduced in the vertebra at an angle and convergingly towards each other, as seen in Fig. 1. In the illustrated embodiment, the first screw 10 is larger than the second screw 20 and defines near its distal end an oblique threaded through opening 12. The two screws 10 and 20 have flat head 14 and 24 which define a depression which is shaped to be engageable by a screwdriver, or the like, for inducing torque thereto and causing the screws 10 and 20 to rotatably engage the vertebra V and gradually advance translationally thereinto, in a conventional manner. In Fig. 1, these depressions are slots 16 and 26 for use with a flat blade screwdriver, although the depressions could be cruciform, square, hexagonal, torx, etc., shaped.

The second screw 20 has a threaded stem of which at least a distal section is smaller than that of the first screw 10 as the second screw 20 is adapted to extend through the opening 12 of the first screw 10 such as to threadably engage the same. Indeed, the males threads of the second screw 20 are designed to engage the female threads of the opening 12 of the first screw 10 thereby securing together the distal ends of the two screws 10 and 20. With these distal ends so engaged and with the screws 10 and 20 extending in a convergent attitude, there is resistance, where the two screws 10 and 20 are engaged together, to the forces which tend to cause the screws to gradually loosen, whereby it is virtually impossible for the screws 10 and 20 to loosen (unless the vertebra V itself is destructed where it is engaged by the screws 10 and 20, or unless one of the screws 10 and 20 breaks).

In fact, the first screw 10 acts as a nut for the second screw 20, and this within the vertebra V itself in Figs. 1 and 2 (as opposed to conventional nuts which normally engage the screw or bolt on the outside of the object through which the screw or bolt extends.

The first screw 10 is preferably provided at its head 14 with indicia (colour, index, etc.) to indicate the position of its distal end so that the position or orientation of its threaded opening 12 can be more easily determined thereby facilitating the introduction of the second screw 20 therethrough. An aiming system may be used as a guide during the screwing process. For instance, to ensure an accurate aiming of the two screws 10 and 20 and their relative engagement, a template may be used to guide both screws from an initial predetermined spacing, along a given plane and along predetermined angles. Alternatively, a neuro-navigation apparatus can also be used, that is a computer software capable of transposing digitised data taken from a pre-surgery medical imagery of the stereotactic space in which the surgeon will operate.

The obliqueness of the threaded opening 12 through the first screw 10 depends on the angle, that is on the spacing on the plate P/P' between the two screws 10 and 20 (i.e. generally the spacing between their heads 14 and 24) in a horizontal plane, as well as the directions of the screws 10 and 20 in the sagittal plane.

The two screws 10 and 20 extend in holes defined in the plate P/P', and would normally have their heads 14 and 24 in abutment with the proximal surface of the plate P/P' (as in Fig. 2, but as opposed to the schematic illustration of Fig. 1 where the heads 14 and 24 are shown spaced from the plate P but simply for illustration purposes). The holes in the plate P/P' are typically angled so as to ensure the crossing of the screws 10 and 20 at a precise location in the vertebra V and so permit the threaded engagement of the second screw 20 in the opening 12 of the first screw 10 once the first screw 10 is completely fixed (i.e. screwed in the vertebra V) and once the position of its opening 12 is determined by way of the indicia on its head 14.

The two screws 10 and 20 and the plate P/P' define a triangular frame (which is well shown in Fig. 1) which is rigid, closed and locked in place, having its components locked together in a solid medium, i.e. the vertebra V, whereby expulsion of the screws 10 and 20 from the vertebra V is opposed. Each of the three components 10, 20 and P/P' of this frame is integral to the preceding component and to the next component. The triangulation screwing process is a concept based on the principle that a frame is much stronger than an open structure. By connecting two screws at their distal ends, it becomes possible to create such a frame. This triangular configuration is also convenient as it allows the anchoring system S, in addition to firmly securing the plate P/P' to the vertebrae V, V₁ and V₂ and preventing a loosening of the plate P/P' with respect to these vertebrae, to extend around the spinal process or canal C and thus around the spinal cord when the plate P/P' is, for instance, installed posteriorly (see Fig. 1).

In the case of the use of the anchoring system S to install the plate P' onto the adjacent vertebrae V₁ and V₂ of the cervical rachis (Fig. 2), along an antero-lateral path, for instance following the removal of an herniated disc, osteosynthesis can be realised by fixing a plate P' (e.g. a "Senegas"-type plate) with anchoring two systems S, as in Fig. 2. The plate P' is, centered about the intersomatic space 30, which is devoid of its natural disc, the latter having been replaced by a disc prosthesis. The first screws 10 are then positioned in the left holes 14 of the plate P', along an antero-posterior axis or slightly obliquely from the left to the right, as the screwdriver will be hindered by the thickness of the oseo-tracheal axis (displaced to the left), before the first screws 10 are screwed through the left holes 14 and into the vertebrae V₁ and V₂. Once the first screws are completely set into the vertebrae V₁ and V₂, and properly positioned using their indicia, the two second screws 20 can be screwed through the right holes 14 of the plate P' and into the vertebrae V₁ and V₂, which is easier than for the first screws 10 as the second screws 20 can be more easily inclined from right to left as the jugulo-carotid bundle is not as obstructive. A scopic control can ensure the proper engagement of the two screws 10 and 20 of each anchoring system S.

In the case of the plate P of Fig. 1 secured posteriorly at least to the vertebra V with the anchoring system S, the determination of the entry points in each of the pedicles of the vertebra V can be realised according to Roy-Camille. The plate P, or a linking rod, is then positioned horizontally and transversely such that its holes are opposite the pre-determined entry points. The screws 10 and 20 are then installed as above to form with the aforementioned triangular frame. This triangular frame, which is rigid and intra-vertebral, can then be solidified to upper and lower frames using plates or rods, in a conventional manner.

In order to facilitate the engagement of the second screw 20 into the first screw 10, the opening 12 in the first screw 10 may, instead of being threaded, have the form of a spherical socket that rotatably accommodates a ball. A hole extends, typically diametrically, through this ball and defines an interior thread, that is a female thread that can be screwably engaged by the male thread of the second screw 20. Therefore, the ball could rotate within the socket to allow for a correction in the direction of the second screw 20 relative to the first screw 10; in other words, if the second screw 20 is slightly off target in its orientation with respect to the hole defined in the ball of the first screw 10, the ball may be slightly rotated to align the longitudinal axis of its hole with the axis of the second screw 20.

It is also contemplated to provide a threadless opening in the first screw 10 instead of the threaded opening 12; in such a case, the opening would be self-tapping in that the male threads of the second screw 20 would tap a thread in the opening of the first screw 10 upon rotary engagement therein. Similarly, the above-mentioned ball could also be threadless and self-tapping. Furthermore, the first screw 10 could be replaced by a threadless pin or nail that would be translationally insertable in the bone and that would define an opening (threaded or self-tapping) at its distal end for receiving the second screw 20.

It is further contemplated to use elongated anchoring members other than the above-described and herein illustrated screws 10 and 20, as well as other means of securing the distal ends of such anchoring members together. For instance, the screws 10 and 20 could be replaced by threadless pins or nails that would be translationally inserted in the bone. In such a case, the distal end of a first one of the anchoring members could define an opening, such as an elongated slot, through which the distal end (which would, for instance, be flat) of a second one of anchoring members could be inserted. A locking mechanism between the two distal ends could take the form of a lateral pin extending from the distal end of the second anchoring member which, after having been passed beyond the elongated slot in the first anchoring member, would be rotated ¼ turn such as to extend behind the body of the first anchoring member thereby locking the distal ends together. Such a pin could be embodied in the distal end of the second anchoring member being L-shaped or T-shaped or defining a barb-shaped extension.

The common feature is two elongated members insertable in the bone and having distal ends capable of being interlocked for preventing unwanted withdrawal of any of the two elongated members from the bone.

Although the present anchoring system S has been shown herein in use to secure a plate P/P' to one or more lumbar (Fig. 1) or cervical (Fig. 2) vertebrae, the system S can also be used to secure rods instead of plates, for instance to the dorso-lumbar rachis, and in fact can be used to affix various objects to various bones of the body, and not only to the rachis. The system S can thus be used not only as described above and herein illustrated, but also in orthopaedic, in neuro-surgical, otorhinolaryngological, maxillo-facial and stomatological applications.

Every component of the anchoring system S is made of a biocompatible material or of a material capable of being so coated.

## Claims

1. An anchoring system for mounting an object to a bone, comprising first (10) and second anchoring (20) members each having proximal and distal ends, said proximal ends being adapted to hold the object to the bone while said distal ends are in the bone;
**characterised in that**;
said proximal ends are spaced from each other and said first and second anchoring members are adapted to be directly connected to each other in the bone.

2. An anchoring system as defined in claim 1, wherein said distal ends define co-operating engagement means for connecting said distal ends together in the bone.

3. An anchoring system as defined in claim 2, wherein said co-operating engagement means comprise inter-engageable male threaded means and female threaded means provided respectively on said distal ends of said second and first anchoring members.

4. An anchoring system as defined in claim 3, wherein said male threaded means comprise a threaded section provided at said distal end of said second anchoring member, and wherein said female threaded means comprise a threaded opening obliquely defined at said distal end of said first anchoring member, said threaded section being adapted to threadably engage said threaded opening.

5. An anchoring system as defined in claim 4, wherein said first arid second anchoring members comprise respectively first and second screws, said first screw defining said threaded opening.

6. An anchoring system as defined in claim 3, wherein said proximal end of said first anchoring member is provided with indicia means for indicating an orientation of said female threaded means in the bone thereby facilitating the engagement therein of said male threaded means of said second anchoring member.

7. An anchoring system as defined in claim 6, wherein said proximal ends of each of said first and second anchoring members are provided with a head engageable by a torque-producing tool for engaging said first and second anchoring members in the bone, said indicia means being at least provided on said head of said first anchoring member.

8. An anchoring system as defined in claim 3, wherein said male threaded means comprise a threaded section provided at said distal end of said second anchoring member, and wherein said female threaded means comprise a threaded opening defined through a ball rotatably mounted in a socket defined at said distal end of said first anchoring member, said threaded section being adapted to threadably engage said threaded opening.

9. An anchoring system as defined in any of the previous claims, further comprising a support member, said proximal ends of said first and second anchoring members being adapted to hold said support member to the bone while said distal ends are connected to each other in the bone thereby forming a triangular frame.

10. An anchoring system as defined in claim 9, wherein said support member defines a pair of holes, said first and second anchoring members being adapted to extend through said holes with said proximal ends engaging said support member adjacent said holes.

11. An anchoring system as defined in claim 10, wherein said first and second anchoring members converge from said proximal ends towards said distal ends, said distal ends defining co-operating engagement means for connecting said distal ends together in the bone.

12. An anchoring system as defined in claim 11, wherein said co-operating engagement means comprise inter-engageable male threaded means and female threaded means provided respectively on said distal ends of said second and first anchoring members.

13. An anchoring system as defined in claim 12, wherein said male threaded means comprise a threaded section provided at said distal end of said second anchoring member, and wherein said female threaded means comprise a threaded opening obliquely defined at said distal end of said first anchoring member, said threaded section being adapted to threadably engage said threaded opening.

14. An anchoring system as defined in claim 13, wherein said first and second anchoring members comprise respectively first and second screws, said first screw defining said threaded opening.

15. An anchoring system as defined in claim 12, wherein said proximal end of said first anchoring member is provided with indicia means for indicating an orientation of said female threaded means in the bone thereby facilitating the engagement therein of said male threaded means of said second anchoring member.

16. An anchoring system as defined in claim 2, wherein said co-operating engagement means comprise inter-engageable male and female means provided respectively on said distal ends of said second and first anchoring members.

17. An anchoring system as defined in claim 16, wherein said female means comprise an elongated slot and said male means include a lateral extension at said distal end of said second anchoring member, said distal end and said lateral extension of said second anchoring member being insertable through said slot, wherein upon adequate rotation of said distal end of said second anchoring member, said lateral extension becomes locked behind said distal end of said first anchoring member.

18. An anchoring system as defined in claim 17, wherein said distal end of said second anchoring member and said lateral extension are one of T-shaped, L-shaped and barb-shaped.

19. An anchoring system as defined in claim 17, wherein said first and second anchoring members are threadless pins or nails.

20. An anchoring system as defined in claim 2, wherein said co-operating engagement means comprise a male threaded means at said distal end of said second anchoring member and an opening in said distal end of said first anchoring member adapted to be tapped by said male threaded means upon rotary engagement of said male threaded means in said opening.

21. An anchoring system as defined in claim 20, wherein said male threaded means comprise a threaded section provided at said distal end of said second anchoring member, and wherein said opening is defined in a ball rotatably mounted in a socket defined at said distal end of said first anchoring member.

## Patentansprüche

1. Verankerungssystem für das Montieren eines Gegenstands an einen Knochen, umfassend erste (10) und zweite Verankerungsglieder (20), die jeweils proximale und distale Enden aufweisen, wobei die proximalen Enden geeignet sind, den Gegenstand am Knochen zu halten, während die distalen Enden sich im Knochen befinden;
**dadurch gekennzeichnet, dass**
die proximalen Enden voneinander im Abstand gehalten werden und die ersten und zweiten Verankerungsglieder geeignet sind, im Knochen direkt miteinander verbunden zu sein.

2. Verankerungssystem wie in Anspruch 1 definiert, wobei die distalen Enden miteinander kooperierende Eingriffsvorrichtungen definieren für das Verbinden der distalen Enden miteinander im Knochen.

3. Verankerungssystem wie in Anspruch 2 definiert, wobei die miteinander kooperierenden Eingriffsvorrichtungen austauschbare männliche Gewindevorrichtungen und weibliche Gewindevorrichtungen umfassen, die jeweils an den distalen Enden der zweiten und ersten Verankerungsglieder bereitgestellt sind.

4. Verankerungssystem wie in Anspruch 3 definiert, wobei jede männliche Gewindevorrichtung einen Gewindeabschnitt umfasst, der am distalen Ende des zweiten Verankerungsglieds bereitgestellt ist, und wobei die weibliche Gewindevorrichtung eine Gewindeöffnung umfasst, die am distalen Ende des ersten Verankerungsglieds schräg definiert ist, wobei der Gewindeabschnitt geeignet ist, die Gewindeöffnung durch das Gewinde in Eingriff zu nehmen.

5. Verankerungssystem wie in Anspruch 4 definiert, wobei die ersten und zweiten Verankerungsglieder jeweils erste und zweite Schrauben umfassen, wobei die erste Schraube die Schraubenöffnung definiert.

6. Verankerungssystem wie in Anspruch 3 definiert, wobei das proximale Ende des ersten Verankerungsglieds mit Indizienvorrichtungen ausgestattet ist zum Anzeigen einer Orientierung der weiblichen Gewindevorrichtung im Knochen, wodurch der Eingriff darin der männlichen Gewindevorrichtung des zweiten Verankerungsglieds ermöglicht wird.

7. Verankerungssystem wie in Anspruch 6 definiert, wobei die proximalen Enden eines jeden der ersten und zweiten Verankerungsglieder mit einem Kopf ausgestattet sind, der durch ein Drehmoment erzeugendes Werkzeug in Eingriff genommen werden kann zum Ineingriffnehmen der ersten und zweiten Verankerungsglieder im Knochen, wobei die Indizienvorrichtung zumindest auf dem Kopf des ersten Verankerungsglieds bereitgestellt ist.

8. Verankerungssystem wie in Anspruch 3 definiert, wobei die männliche Gewindevorrichtung einen Gewindeabschnitt umfasst, der am distalen Ende des zweiten Verankerungsglieds bereitgestellt ist, und wobei die weibliche Gewindevorrichtung eine Gewindeöffnung umfasst, die durch eine Kugel definiert ist, die drehbar in einer Buchse montiert ist, die am distalen Ende der ersten Verankerungsvorrichtung definiert ist und der Gewindeabschnitt geeignet ist, die Gewindeöffnung durch das Gewinde in Eingriff zu nehmen.

9. Verankerungssystem wie in einem der vorhergehenden Ansprüche definiert, umfassend des Weiteren ein Stützglied, wobei die proximalen Enden der ersten und zweiten Verankerungsglieder geeignet sind, das Stützglied am Knochen zu halten, während die distalen Enden im Kochen unter Bildung eines dreieckigen Rahmens miteinander verbunden sind.

10. Verankerungssystem wie in Anspruch 9 definiert, wobei das Stützglied ein Paar Löcher definiert, wobei die ersten und zweiten Verankerungsglieder geeignet sind, sich durch die Löcher hindurch zu erstrecken, wobei die proximalen Enden in das Stützglied neben den Löchern eingreifen.

11. Verankerungssystem wie in Anspruch 10 definiert, wobei die ersten und zweiten Verankerungsglieder von den proximalen Enden auf die distalen Enden zu konvergieren, wobei die distalen Enden kooperierende Vorrichtungen für das Verbinden der distalen Enden miteinander im Knochen definieren.

12. Verankerungssystem wie in Anspruch 11 definiert, wobei die kooperierenden Eingriffvorrichtungen austauschbare männliche und weibliche Gewindevorrichtungen, die jeweils an den distalen Enden der zweiten und ersten Verankerungsglieder bereitstellt sind, umfassen.

13. Verankerungssystem wie in Anspruch 12 definiert, wobei die männlichen Gewindevorrichtungen einen Gewindeabschnitt umfassen, der am distalen Ende des zweiten Verankerungsglieds bereitgestellt ist, und wobei die weiblichen Gewindevorrichtungen eine Gewindeöffnung umfassen, die am distalen Ende des ersten Verankerungsglieds schräg definiert ist, wobei der Gewindeabschnitt geeignet ist, die Gewindeöffnung durch das Gewinde in Eingriff zu nehmen.

14. Verankerungssystem wie in Anspruch 13 definiert, wobei die ersten und zweiten Verankerungsglieder jeweils erste und zweite Schrauben umfassen, wobei die erste Schraube die Schraubenöffnung definiert.

15. Verankerungssystem wie in Anspruch 12 definiert, wobei das proximale Ende des ersten Verankerungsglieds mit Indizienvorrichtungen ausgestattet ist zum Anzeigen einer Orientierung der weiblichen Gewindevorrichtung im Knochen, wodurch der Eingriff darin der männlichen Gewindevorrichtung des zweiten Verankerungsglieds ermöglicht wird.

16. Verankerungssystem wie in Anspruch 2 definiert, wobei die miteinander kooperierenden Eingriffsvorrichtungen austauschbare männliche und weibliche Vorrichtungen umfassen, die jeweils an den distalen Enden der zweiten und ersten Verankerungsglieder bereitgestellt sind.

17. Verankerungssystem wie in Anspruch 16 definiert, wobei die weiblichen Vorrichtungen einen längsgezogenen Schlitz umfassen und die männlichen Vorrichtungen eine seitliche Verlängerung am distalen Ende des zweiten Verankerungsglieds enthalten, wobei das distale Ende und die seitliche Verlängerung des zweiten Verankerungsglieds durch den Schlitz einsetzbar sind, wobei auf ausreichendes Drehen des distalen Endes des zweiten Verankerungsglieds hin die seitliche Verlängerung hinter dem distalen Ende des ersten Verankerungsglieds verriegelt wird.

18. Verankerungssystem wie in Anspruch 17 definiert, wobei das distale Ende des zweiten Verankerungsglieds und die seitliche Verlängerung eines unter T-förmigen, L-förmigen und domförmigen sind.

19. Verankerungssystem wie in Anspruch 17 definiert, wobei die ersten und zweiten Verankerungsglieder gewindelose Stifte oder Nägel sind.

20. Verankerungssystem wie in Anspruch 2 definiert, wobei die miteinander kooperierenden Eingriffsvorrichtungen eine männliche Gewindevorrichtung am distalen Ende des zweiten Verankerungsglieds und eine Öffnung im distalen Ende des ersten Verankerungsglieds umfassen, die geeignet ist, durch die männliche Gewindevorrichtung auf den Dreheingriff der männlichen Gewindevorrichtung hin angeschlagen zu werden.

21. Verankerungssystem wie in Anspruch 20 definiert, wobei die männliche Gewindevvnichtung einen Gewindeabschnitt umfasst, der am distalen Ende des zweiten Verankerungsglieds bereitgestellt ist, und wobei Öffnung durch eine Kugel definiert ist, die drehbar in einer Buchse montiert ist, die am distalen Ende der ersten Verankerungsvorrichtung definiert ist.

## Revendications

1. Système d'ancrage pour monter un objet sur un os, comprenant des premier (10) et deuxième (20) éléments d'ancrage comportant chacun des extrémités proximales et distales, lesdites extrémités proximales pouvant retenir l'objet à l'os tandis que lesdites extrémités distales se trouvent dans l'os,
**caractérisé en ce que**
lesdites extrémités proximales sont espacées l'une de l'autre et lesdits premier et deuxième éléments d'ancrage peuvent être reliés directement l'un à l'autre dans l'os.

2. Système d'ancrage suivant la revendication 1, dans lequel lesdites extrémités distales définissent des moyens de prise correspondants pour relier lesdites extrémités distales l'une à l'autre dans l'os.

3. Système d'ancrage suivant la revendication 2, dans lequel lesdits moyens de prise correspondants comprennent des moyens filetés mâles et des moyens filetés femelles pouvant aller en prise les uns avec les autres disposés respectivement sur lesdites extrémités distales desdits deuxième et premier éléments d'ancrage.

4. Système d'ancrage suivant la revendication 3, dans lequel lesdits moyens filetés mâles comprennent une section filetée disposée au niveau de ladite extrémité distale dudit deuxième élément d'ancrage, et dans lequel lesdits moyens filetés femelles comprennent une ouverture filetée définie obliquement au niveau de ladite extrémité distale dudit premier élément d'ancrage, ladite section filetée pouvant venir en prise par filetage avec ladite ouverture filetée.

5. Système d'ancrage suivant la revendication 4, dans lequel lesdits premier et deuxième éléments d'ancrage comprennent respectivement des première et deuxième vis, ladite première vis définissant ladite ouverture filetée.

6. Système d'ancrage suivant la revendication 3, dans lequel ladite extrémité proximale dudit premier élément d'ancrage est pourvue de moyens indicateurs pour indiquer une orientation desdits moyens filetés femelles dans l'os, facilitant de ce fait la prise dans ceux-ci desdits moyens filetés mâles dudit deuxième élément d'ancrage.

7. Système d'ancrage suivant la revendication 6, dans lequel lesdites extrémités proximales de chacun desdits premier et deuxième éléments d'ancrage sont pourvues d'une tête pouvant aller en prise avec un outil générateur de couple pour mettre en prise lesdits premier et deuxième éléments d'ancrage dans l'os, lesdits moyens indicateurs étant au moins disposés sur ladite tête dudit premier élément d'ancrage.

8. Système d'ancrage suivant la revendication 3, dans lequel lesdits moyens filetés mâles comprennent une section filetée disposée au niveau de ladite extrémité distale dudit deuxième élément d'ancrage, et dans lequel lesdits moyens filetés femelles comprennent une ouverture filetée définie dans une bille montée de manière rotative dans une cavité définie au niveau de ladite extrémité distale dudit premier élément d'ancrage, ladite section filetée pouvant venir en prise par filetage avec ladite ouverture filetée.

9. Système d'ancrage suivant l'une quelconque des revendications précédentes, comprenant en outre un élément de support, lesdites extrémités proximales desdits premier et deuxième éléments d'ancrage pouvant retenir ledit élément de support à l'os tandis que lesdites extrémités distales sont reliées l'une à l'autre dans l'os, formant de ce fait un cadre triangulaire.

10. Système d'ancrage suivant la revendication 9, dans lequel ledit élément de support définit deux orifices, lesdits premier et deuxième éléments d'ancrage pouvant s'étendre à travers lesdits orifices, lesdites extrémités proximales venant en prise avec ledit élément de support à proximité desdits orifices.

11. Système d'ancrage suivant la revendication 10, dans lequel lesdits premier et deuxième éléments d'ancrage convergent desdites extrémités proximales vers lesdites extrémités distales, lesdites extrémités distales définissant des moyens de prise correspondants pour relier lesdites extrémités distales l'une à l'autre dans l'os.

12. Système d'ancrage suivant la revendication 11, dans lequel lesdits moyens de prise correspondants comprennent des moyens filetés mâles et des moyens filetés femelles pouvant aller en prise les uns avec les autres disposés respectivement sur lesdites extrémités distales desdits deuxième et premier éléments d'ancrage.

13. Système d'ancrage suivant la revendication 12, dans lequel lesdits moyens filetés mâles comprennent une section filetée disposée au niveau de ladite extrémité distale dudit deuxième élément d'ancrage, et dans lequel lesdits moyens filetés femelles comprennent une ouverture filetée définie obliquement au niveau de ladite extrémité distale dudit premier élément d'ancrage, ladite section filetée pouvant venir en prise par filetage avec ladite ouverture filetée.

14. Système d'ancrage suivant la revendication 13, dans lequel lesdits premier et deuxième éléments d'ancrage comprennent respectivement des première et deuxième vis, ladite première vis définissant ladite ouverture filetée.

15. Système d'ancrage suivant la revendication 12, dans lequel ladite extrémité proximale dudit premier élément d'ancrage est pourvue de moyens indicateurs pour indiquer une orientation desdits moyens filetés femelles dans l'os, facilitant de ce fait la prise dans ceux-ci desdits moyens filetés mâles dudit deuxième élément d'ancrage.

16. Système d'ancrage suivant la revendication 2, dans lequel lesdits moyens de prise correspondants comprennent des moyens mâles et femelles pouvant aller en prise les uns avec les autres disposés respectivement sur lesdites extrémités distales desdits deuxième et premier éléments d'ancrage.

17. Système d'ancrage suivant la revendication 16, dans lequel lesdits moyens femelles comprennent une rainure allongée et lesdits moyens mâles comprennent une extension latérale au niveau de ladite extrémité distale dudit deuxième moyen d'ancrage, ladite extrémité distale et ladite extension latérale dudit deuxième élément d'ancrage pouvant être insérées dans ladite rainure, dans lequel, moyennant une rotation adéquate de ladite extrémité distale dudit deuxième élément d'ancrage, ladite extension latérale se verrouille derrière ladite extrémité distale dudit premier élément d'ancrage.

18. Système d'ancrage suivant la revendication 17, dans lequel ladite extrémité distale dudit deuxième élément d'ancrage et ladite extension latérale ont la forme d'un T, d'un L ou d'une barbelure.

19. Système d'ancrage suivant la revendication 17, dans lequel lesdits premier et deuxième éléments d'ancrage sont des clous ou des broches sans filet.

20. Système d'ancrage suivant la revendication 2, dans lequel lesdits moyens de prise correspondants comprennent des moyens filetés mâles au niveau de ladite extrémité distale dudit deuxième élément d'ancrage et une ouverture dans ladite extrémité distale dudit premier élément d'ancrage dans laquelle peuvent être taraudés lesdits moyens filetés mâles lors de la prise par rotation desdits moyens filetés mâles dans ladite ouverture.

21. Système d'ancrage suivant la revendication 20, dans lequel lesdits moyens filetés mâles comprennent une section filetée disposée au niveau de ladite extrémité distale dudit deuxième élément d'ancrage, et dans lequel ladite ouverture est définie dans une bille montée en rotation dans une cavité définie au niveau de ladite extrémité distale dudit premier élément d'ancrage.
